# EUROPEAN PATENT SPECIFICATION

(11) **EP 3 716 865 B1**
(45) Date of publication and mention of the grant of the patent: **01.11.2023**
(21) Application number: 17933461.0
(22) Date of filing: 01.12.2017
(51) Int. Cl.: A61B 17/04, A61B 17/58, A61B 17/68, A61B 17/84, A61B 17/56

(54) **EXTRA JOINT STABILIZATION CONSTRUCT**
ZUSÄTZLICHES GELENKSTABILISIERUNGSKONSTRUKT
CONSTRUCTION DE STABILISATION D'ARTICULATION SUPPLÉMENTAIRE

(43) Date of publication of application: 07.10.2020
(73) Proprietor: Crossroads Extremity Systems, LLC, Memphis, TN 38119 (US)
(72) Inventor: FALLIN, T. Wade, Hyde Park, Utah 84318 (US); TABER, Justin, Honolulu, HI 96822 (US)
(74) Representative: Carpmaels & Ransford LLP
(86) International application number: PCT/US2017/064178
(87) International publication number: WO 2019/108224

(56) References cited:
- WO-A1-2009/055800
- US-A1- 2009 157 124
- US-A1- 2010 262 185
- US-A1- 2010 262 185
- US-A1- 2014 277 128
- US-A1- 2015 173 739
- US-A1- 2016 374 661
- US-A1- 2017 071 592
- US-B2- 9 131 937

## Description

### Background

Ligaments interconnect bones of the skeletal system and are involved with the stabilization and kinematics of skeletal joints. Various injuries may occur that result in compromised ligament function and/or bone fractures. Such injuries include, for example, partial and complete tears and avulsion of the bone where a ligament attaches to a bone. Such injuries occur throughout the skeletal system.

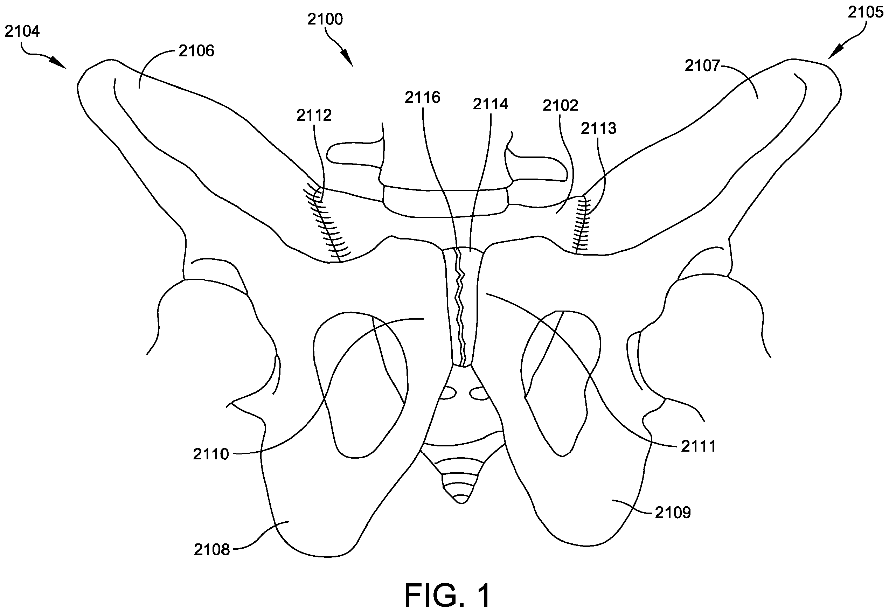

By way of example, the human pelvis 2100 is a complex junction of multiple bones and soft tissues, as shown in FIG. 1. The sacrum 2102 bounds the posterior aspect of the pelvis with a pair of hip bones 2104, 2105 bounding the lateral and anterior aspects of the pelvis. Each hip bone is composed of three parts including the ilium 2106, 2107; ischium 2108, 2109; and pubis 2110, 2111. The sacrum is joined to each hip bone 2104, 2105 by strong ligaments at the sacroiliac joint 2112, 2113. The hip bones 2104, 2105 are joined anteriorly at the cartilaginous pubic symphysis 2114.

Various conditions may cause the pelvis to become unstable. For example, childbirth and traumatic injury may result in instability at the sacroiliac joint 2112, 2113 and/or the pubic symphysis 2114. For example, a traumatic anterior-posterior compression fracture may result in a separation 2116 between the hip bones at the pubic symphysis 2114, as shown in FIG. 1, and loosening of the sacroiliac joint 2112, 2113 leading to pelvic instability.

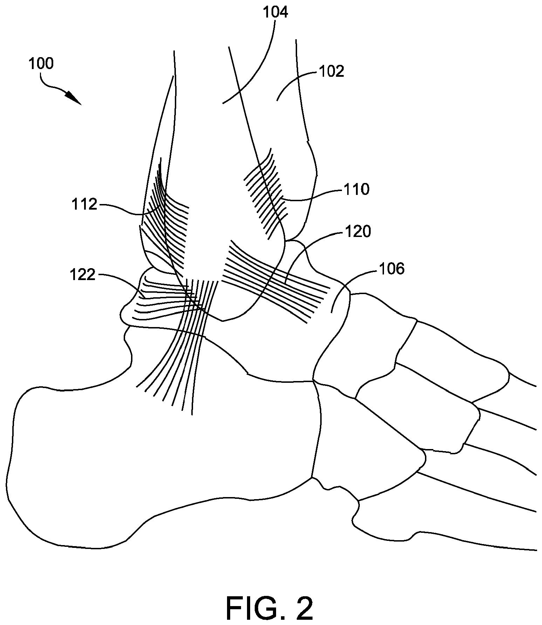

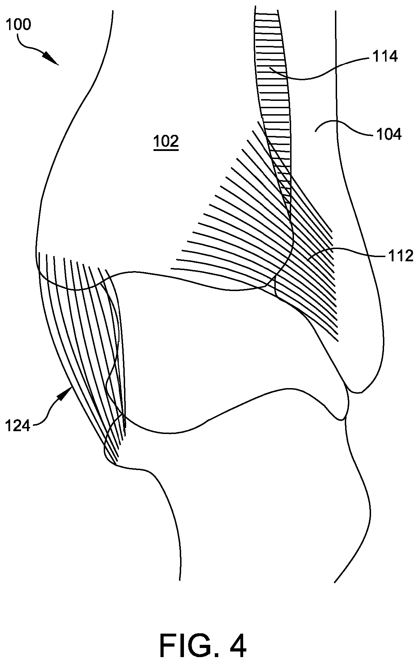

In another example, the human ankle 100 is a complex junction of multiple bones and soft tissues, as shown in FIGS. 2-4. The ankle includes joints between the tibia 102, fibula 104, and talus 106. The joint between the tibia 102 and fibula 104 is a syndesmosis or slightly movable joint in which the bones are joined together by connective tissue. The syndesmosis between the tibia and fibula includes the anterior inferior tibiofibular ligament (AITFL) 110, the posterior inferior tibiofibular ligament (PITFL) 112, and the interosseous ligament (IOL) 114 (FIG. 4). The syndesmosis ligaments are often injured in high ankle sprains. Other injury prone ligaments of the ankle joint include, among others, the anterior talofibular ligament (ATFL) 120, the posterior talofibular ligament (PTFL) 122 and the deltoid ligament complex 124 including superficial and deep deltoid ligaments.

What is needed is improved implants, instruments and methods to stabilize bone fractures and/or reinforce ligaments.

WO 2009/055800 discloses an anchor assembly including an anchor defining a proximal portion, a distal portion, a cavity and an opening to the cavity and an insertion member configured for arrangement within the anchor cavity.

US 2009/157124 discloses an anchor assembly including an opening to the cavity and an insertion member including a body having a proximal end portion and a flat distal end portion, and a head coupled to the proximal end portion of the body.

US 2010/262185 discloses a knotless aperture fixation system, and method for use thereof, for securing a flexible material to a nearby tissue using knotless fixation achieved by a compression fit of the flexible material between two smooth surfaces.

### Summary

This Summary is provided to introduce a selection of concepts in a simplified form that are further described below in the Detailed Description. This Summary is not intended to identify key aspects or essential aspects of the claimed subject matter. Moreover, this Summary is not intended for use as an aid in determining the scope of the claimed subject matter.

One embodiment provides a knotless returning and locking system for bone fracture stabilization and soft tissue repair and reinforcement according to claim 1. Further developments of the invention can be gathered from the dependent claims.

Additional objects, advantages and novel features of the technology will be set forth in part in the description which follows, and in part will become more apparent to those skilled in the art upon examination of the following, or may be learned from practice of the technology.

### Brief Description of the Drawings

Non-limiting and non-exhaustive embodiments of the present invention, including the preferred embodiment, are described with reference to the following figures, wherein like reference numerals refer to like parts throughout the various views unless otherwise specified. Illustrative embodiments of the invention are illustrated in the drawings, in which:
FIG. 1 illustrates an anterior view of a human pelvis having an anterior-posterior compression fracture;
FIGURE 2 illustrates a right view of a human ankle joint;
FIGURE 3 illustrates a front view of a human ankle joint;
FIGURE 4 illustrates a rear view of a human ankle joint;
FIGURES 5-7 illustrate respective top, front, and cross-sectional views of one embodiment of a returning and locking anchor;
FIGURE 8 illustrates a cross-sectional view of a receiver of a suture locking feature of the suture returning and locking anchor of FIGURES 5-7;
FIGURE 9 illustrates a cross-sectional view of the receiver of FIGURE 8 having a set screw inserted therein to form an interference fit between a suture and the receiver and the set screw;
FIGURE 10 illustrates a front view of the set screw of FIGURE 9 without threading;
FIGURE 11 illustrates the cross-sectional view of the set screw and the receiver of FIGURE 9, without threading;
FIGURES 12-14 illustrate respective exploded, front, and cross-sectional views of another embodiment of a suture returning and locking anchor;
FIGURES 15-18 illustrate respective side, perspective-exploded, and cross-sectional views of one embodiment of an anchor driver engaging with the returning and locking anchor of FIGURES 5-7;
FIGURES 19-22 show partial sectional anterior views of the pelvis of FIG. 1 and illustrate the steps of an operative sequence for stabilizing the compression fracture of FIG. 1 using embodiments of the disclosed devices;
FIGURE 23 provides a flowchart detailing the operative sequence illustrated by FIGURES 19-22;
FIGURE 24 illustrates exemplary reinforcement constructs for the deltoid ligament complex and the posterior inferior tibiofibular ligament (PITFL)/anterior inferior tibiofibular ligament (AITFL) according to embodiments of the disclosed devices;
FIGURE 25 illustrates one embodiment of a ligament reinforcement construct using a single suture returning and locking anchor to reinforce two separate ligaments according to embodiments of the disclosed devices; and
FIGURE 26 illustrates one embodiment of an attachment construct for soft tissue repair or tendon reattachment according to embodiments of the disclosed devices.

### Detailed Description

Embodiments are described more fully below in sufficient detail to enable those skilled in the art to practice the system and method. However, embodiments may be implemented in many different forms and should not be construed as being limited to the embodiments set forth herein. The following detailed description is, therefore, not to be taken in a limiting sense.

The technology discussed herein relates to apparatus and corresponding methods of use for preparing ligament reinforcement and bone fracture repair constructs. Embodiments include a number of suture returning and locking anchors, anchor drivers, and extra joint ligament reinforcement and/or bone fracture repair constructs constructed via operative methods employing the devices and instruments described herein.

### Combined Suture Returning and Locking Anchors

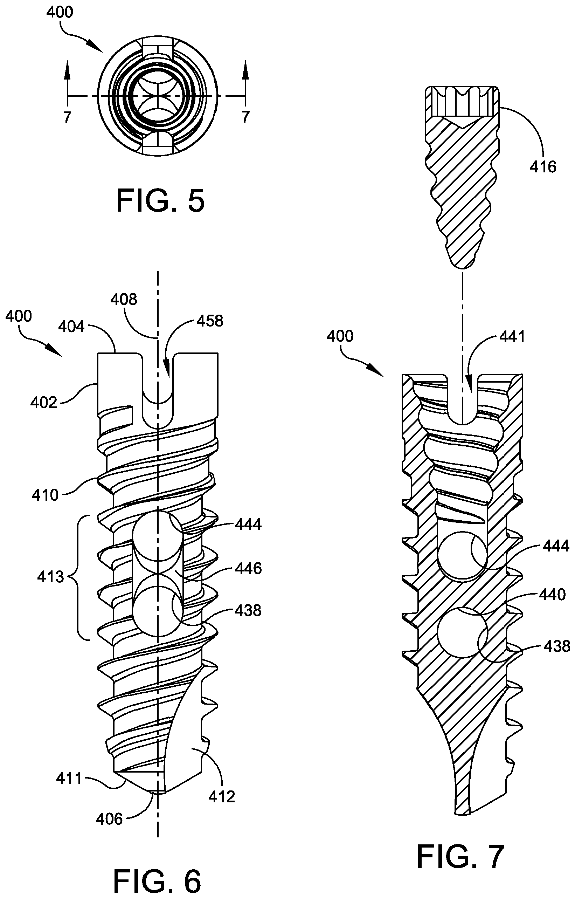

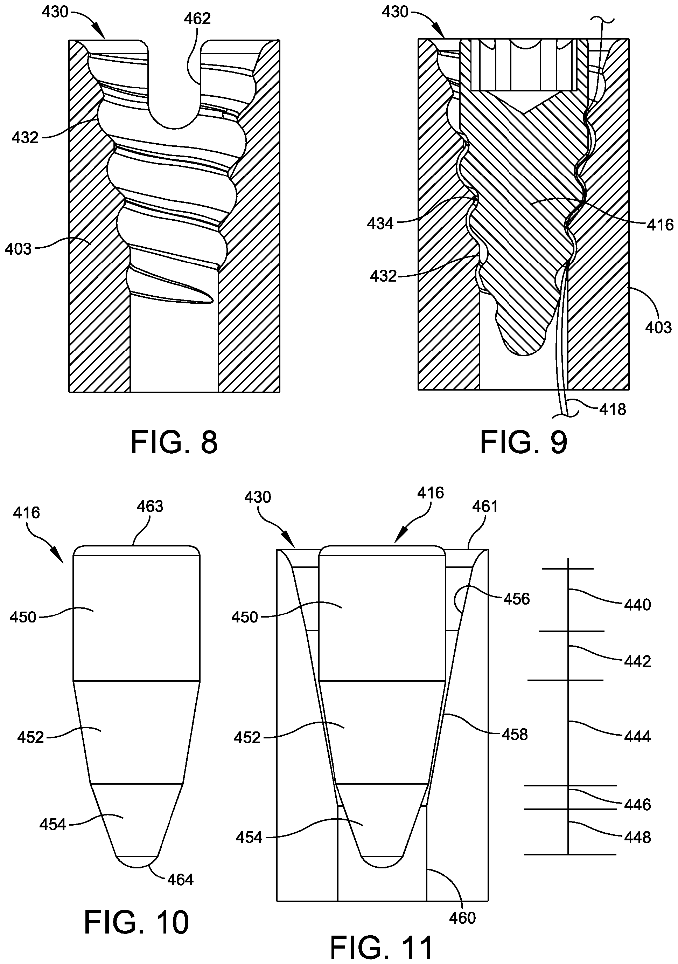

FIGS. 5-7 illustrate top, front, and cross-sectional exploded views of one embodiment of a suture returning and locking anchor 400. In FIGS. 5-7, the anchor 400 includes an anchor body 402 having a proximal end 404, a distal end 406, and defining a longitudinal axis 408. An external bone thread 410 extends around most of the body 402 except proximally where the thread runs out distal to the proximal end, and distally where the thread runs out at a tapered tip 411. The bone thread 410 includes a self-tapping flute 412 at the distal end. A set screw 416 is configured for rotational insertion and locking within the proximal end 404, as detailed further in relation to FIGS. 8-11 below.

The anchor 400 includes a suture return feature 413. In more detail and in this embodiment, the body 402 includes a first transverse hole 438 forming opposed openings on opposite sides of the body 402 through which a suture may be threaded to provide a suture return. While the external bone thread 410 is engaged in a bone, a flexible synthetic strand such as, for example, a suture, a suture tape, a cable or another suitable flexible synthetic strand (hereinafter a "flexible strand," "flexible synthetic strand," or "suture") may be pulled against a proximal margin 440 of the hole 438 to allow the suture to be tensioned. The body further includes an axial hole or passage 441 extending from an opening at the proximal end 404 toward the distal end 406. A second transverse hole 444 extends through the body 402 to form opposed openings on opposite sides of the body 402. The second transverse hole 444 is offset proximally from the first transverse hole 438 and communicates with the axial hole 441.

In use, a suture may be threaded into the axial hole 441 from the proximal end 404, out one of the openings of the second transverse hole 444, through the first transverse hole 438, in the other of the openings of the second transverse hole 444, and out the axial hole 441 so that the suture is routed about the proximal margin 440 within the proximal portion of the body 402.

The anchor body 402 may further contain relief grooves 446 connecting the openings of the first and second transverse holes 438, 444 on each side of the body 402. The relief grooves 446 allow the suture to pass from the axial hole 441 to the first transverse hole 438 while projecting less, or not at all, from the sides of the body 402 to protect the suture from abrasion and to allow the suture to slide more easily while it is being routed and tensioned. In this embodiment, the body 402 provides a tubular extension into a bone to protect the suture from abrasion from the bone as well as to protect the bone from abrasion or cutting from the suture.

The anchor 400 also includes a suture locking feature detailed in the example of FIGS. 8-11. FIG. 8 provides an enlarged view of the internal features of a proximal portion 403 of the body 402, and FIG. 9 provides a cross-sectional view of the proximal portion 403 of the body 402 in receipt of the set screw 416. In this embodiment, the proximal portion 403 of the body 402 includes a receiver 430 having a tapered receiver thread 432, and the set screw 416 has a tapered external thread 434. Both the receiver thread 432 and the set screw thread 434 are rounded knuckle threads. In addition, the receiver 430/receiver thread 432 and the set screw 416/set screw thread 434 feature multiple discrete taper angles that transition proximally to distally to provide for progressive gripping and releasing of the suture 418 to provide a strong grip on the suture while reducing the risk of suture damage or severing.

To address the taper angles in greater detail, FIGS. 10-11 illustrate the set screw 416 and the receiver 430 without their knuckle threading to better illustrate the gradual transitions of their tapers. In this embodiment, the set screw 416 is cylindrical at a proximal portion 450, has a relatively small angled taper over its mid portion 452, and has a relatively large angled taper over its distal portion 454 which terminates in a rounded tip 464. The receiver 430 has a relatively large angled taper at a proximal portion 456, has a relatively small angled taper over its mid portion 458, and is cylindrical at its distal portion 460. When the set screw 416 and the receiver 430 are mated, they provide progressively less clearance between them from the proximal end of the anchor 400 to their mid portions and progressively more clearance between them from their mid portions distally to the end of the set screw 416.

This opposing tapered configuration of the set screw 416 versus the receiver 430 incorporates the principal of the Morse taper for mating components. That is, the opposing conical shapes of the set screw 416 and the receiver 430 are closely matched in angle at their mid portions 452, 458, causing the respective surfaces of the set screw 416 and the receiver 430 to achieve an interference fit about the suture 418 over the mid portions 452, 458 of the set screw 416 and the receiver 430, with gradual transitions proximally leading into and distally leading out of the interference fit. This gradual transition of compression forces applied to the suture 418 disposed between the set screw 416 and the receiver 430 leads to an enhancement in suture fixation/locking strength, and simultaneously reduces the risk of severing the suture 418 that is present with greater magnitudes of compression force transition.

In one embodiment, the mid portions 452, 458 of the set screw 416 and the receiver 430 are of the same length and aligned. In this embodiment, there are three zones or amounts of clearance between the set screw 416 and the receiver 430 progressing in three steps from a relatively large amount of clearance proximally to a relatively small amount of clearance over their mid portions to a relatively large amount of clearance distally.

Alternatively, and as shown in the example of FIGS. 8-11, the set screw 416 can be driven so that the beginning of its mid portion 452 is positioned distal of the beginning of the receiver mid portion 458, and the end of the set screw mid portion 452 is positioned proximal of the end of the receiver mid portion 458, as shown in FIG. 11. This arrangement results in five clearance zones 440, 442, 444, 446, and 448 for an even more gradual progression of gripping and releasing of the suture 418. Any number of taper angle steps may be provided on the set screw 416 and the receiver 430, and any arrangement of overlap or radius blending may be provided to produce any number of progressive clearance steps to transition proximally to distally from no grip to maximum grip to no grip on the suture 418, protecting the suture through the gradual increase and decrease of stress placed on the suture 418.

Referring to FIG. 11, the first zone 440 provides the most clearance proximally and the clearance decreases distally at the angular difference between the cylindrical proximal portion 450 of the set screw 416 and the relatively larger angle of the proximal portion 456 of the receiver 430. The second zone 442 clearance decreases distally at the angular difference between the cylindrical proximal portion 450 of the set screw 416 and the relatively smaller angle of the mid portion 458 of the receiver 430. The third zone 444 provides the least clearance and corresponds to where the mid portions 452, 458 of the set screw 416 and the receiver 430 coincide. The fourth zone 446 clearance increases distally at the angular difference between the relatively smaller angle of the mid portion 458 of the receiver 430 and the relatively larger angle of the distal portion 454 of the set screw 316. The fifth zone 348 provides the most clearance distally and the clearance increases distally at the angular difference between the relatively larger angle of the distal portion 454 of the set screw 316 and the cylindrical portion 460 of the receiver 330.

In the illustrative example of FIGS. 8-11, the set screw 416 taper is cylindrical in the first proximal portion 450, 10 degrees per side in the second mid portion 452, and 20 degrees per side in the distal portion 454. The receiver 430 taper is 40 degrees per side in the first proximal portion 456, 10 degrees per side in a second mid portion 458, and cylindrical at a third distal portion 460. The resulting relief tapers corresponding to the five zones 440, 442, 444, 446, 448 illustrated in FIG. 11, proximally to distally, are 20 degrees, 10 degrees, 0 degrees, 10 degrees, and 20 degrees. In this embodiment, the proximal ends 461, 463 of the receiver 430 and the set screw 416 are chamfered and the distal end 464 of the set screw 416 is rounded to further eliminate any sharp edges to further smooth the path of the suture and to provide easier starting of the screw.

While the embodiment of the suture locking feature of FIGS. 8-11 features opposing tapers on the set screw 416 and the receiver 430, it should be understood that the invention contemplates any appropriate tapering configuration that provides a gradual increase and decrease of compression forces applied, proximally to distally, to the interference fit of the suture 418 between the set screw 416 and the receiver 430. For example, the set screw 416 may be entirely cylindrical through its proximal, mid, and distal portions, maintaining the above described configuration of the receiver 430. In another example, the proximal, mid, and distal portions 450, 452, 454 of the set screw 416 may be angled to form an egg-like or football shape, while the proximal, mid, and distal portions 456, 458, 460 of the receiver 430 remain cylindrical.

The locking feature discussed in relation to FIGS. 8-11 provides both a knotless and reversible mechanism for locking out the suture 418 relative to the returning and locking anchor 400. Because an interference fit between the suture 418, the set screw 416, and the receiver 430 provides the compression force required to secure the suture 418 in tension relative to the anchor 400, the locking feature provides a knotless fixation, thereby reducing the probability of bone and/or tissue abrasion and/or aggravation that is often caused by knotted fixations. Moreover, because the locking mechanism protects the integrity of the suture through the gradual increase and decrease of stress placed on the suture 418, discussed above, the knotless fixation is truly reversible in that the set screw 416 may be rotationally inserted to lock out the suture 418 relative to the anchor 400 without damaging the suture 418 and/or risking its structural integrity. As a result, a surgeon may lock and unlock the suture 418 relative to the anchor 400 multiple times to achieve an optimal fixation while maintaining confidence in the quality of the ultimate knotless fixation.

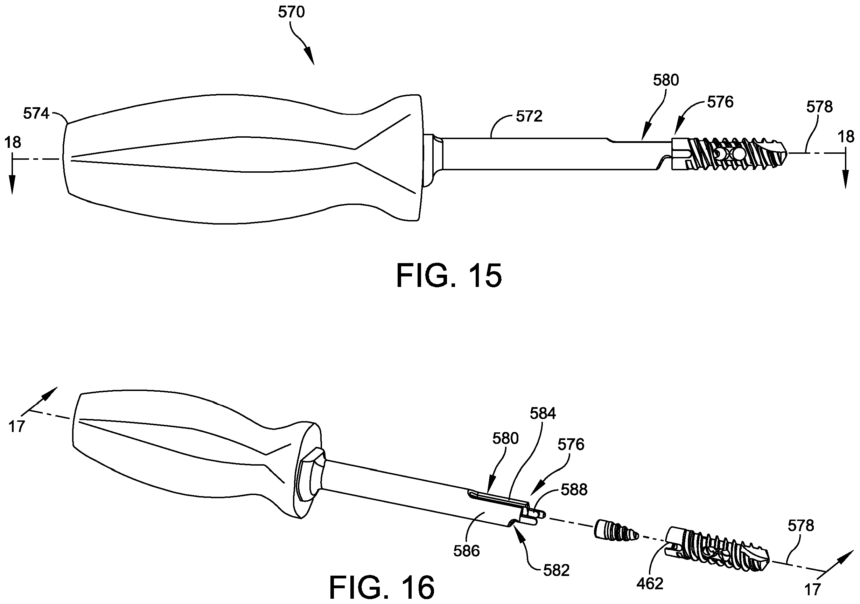

The body 402 of the suture returning and locking anchor 400 further includes a driver feature 458 in the form of opposing slots 462 (FIG. 16) formed in the proximal wall of the body 402 for receiving an anchor driver such that the anchor driver does not block the receiver 430 from receiving the set screw 416 and for providing counter-torque stabilization such that a set screw driver does not axially shift the body 402 during insertion of the set screw 416, as discussed further below in relation to 15-18.

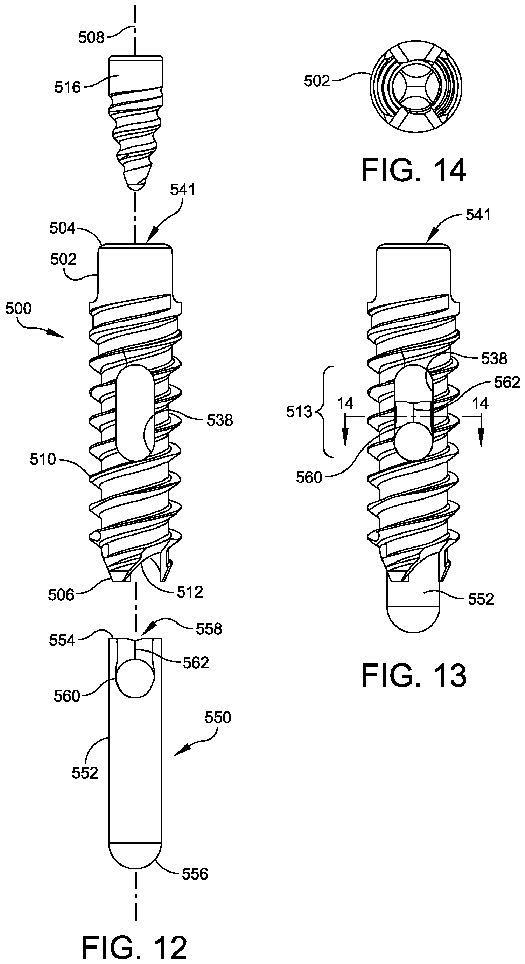

FIGS. 12-14 illustrate exploded, front, and cross-sectional views of another example of a suture returning and locking anchor 500 that is similar to the anchor 400. However, the anchor 500 of FIGS. 12-14 is a two-piece anchor having an axial through-hole 541 that extends through an entirety of a body 502 of the anchor 500.

In this embodiment, the anchor 500 includes the anchor body 502 having a proximal end 504, a distal end 506, and defining a longitudinal axis 508. An external bone thread 510 extends around most of the body 502 except proximally where the thread runs out. The bone thread 510 includes a self-tapping flute 512 at the distal end. A set screw 516 is configured for rotational insertion and locking according to the locking arrangement discussed above in relation to FIGS. 8-11.

The anchor 500 may provide a suture return function either internally or externally. In an internal configuration, the anchor 500 may incorporate a removable suture return insert 550. The suture return insert 550 includes a longitudinal body 552 centered about the longitudinal axis 508 and sized to press fit within the axial hole 541 extending through the anchor body 502. The suture return insert 550 has a proximal end 554, a closed distal end 556, and an axial hole or passage 558 extending distally from the proximal end 554. A first transverse hole 560 forms opposed openings on opposite sides of the insert body 552. The first transverse hole 560 communicates with the axial hole 558 of the insert body 552 such that when the proximal end 554 of the insert 500 is inserted into the distal end 506 of the anchor body 502, the first transverse hole 560 within the insert body 552 aligns with a distal portion of a second transverse hole 538 within the anchor body 502 to form an internal suture return feature 513, as shown in FIG. 13, which is similar to the return feature 413 discussed above in relation to the anchor 400 of FIGS. 5-7.

In use in the internal configuration, a suture may be threaded into the axial hole 541 of the anchor 500 from the proximal end, out one of the openings of the second transverse hole 538 in the anchor body 502, through the first transverse hole 560 in the insert 550, in the other of the openings of the second transverse hole 538, and out the communicating axial holes 441, 558 of the anchor body 402 and the insert body 552 such that the suture is routed within the proximal portion of the anchor 500. The insert body 552 may further contain relief grooves 562 connecting the openings of the first and second transverse holes 560, 538 on each side of the anchor 500, when the insert 550 is disposed within the anchor body 502. The relief grooves 562 allow the suture to pass from the axial holes 541, 558 to the first transverse hole 560 while projecting less, or not at all, from the sides of the anchor body 502 to protect the suture from abrasion and to allow the suture to slide more easily while it is being routed and tensioned.

In an external configuration, the return insert 550 is excluded and the suture return is formed by either the second transverse hole 538 or the distal end 506 of the anchor body 502. In use in the external return configuration, the suture enters the axial hole 541 at the proximal end 504 of the anchor 500 and exits the axial hole 541 at either the second transverse hole 538 or the distal end of the anchor 500, with a return suture path outside the anchor body 502. In various embodiments, the return suture path passes through a notch or recess formed in the outer wall of the anchor body, through a relief groove in the bone that projects radially from the bone tunnel. Alternatively, the suture may exit the distal end 506 of the anchor and continue on a path directly to another fixation point (e.g., continue along the longitudinal axis 508, without a return, within a bone tunnel to another locking anchor).

### Counter-Torque Anchor Driver

FIGS. 15-18 illustrate an example of an anchor driver 570 for use with, for example, the anchor 400 of FIGS. 5-7. The anchor driver includes an elongated body 572 extending from a proximal end 574 to a distal end 576 and defining a longitudinal axis 578. The distal end 576 of the driver includes opposed clearance slots 580, 582 opening distally and defining spaced apart driver legs 584, 586. The distal ends of the driver legs 584, 586 form opposing tabs 588 that engage the opposed slots 462 of the anchor 400. The driver tabs 588 and driver slots 580, 582 are sized so that with the tabs 588 fully engaged in the slots 462 of the anchor, the driver slots 580, 582 provide clearance 592, 594 between the driver 570 and the anchor 400 for the suture 418.

A portion of each tab 588 abuts the proximal end of the anchor 400 and provides a distal facing bearing surface to resist forces (e.g., torsional forces) generated when tensioning the suture 418 via a rotational input from a set screw driver (not shown) to the set screw 416 to engage the locking mechanism discussed above in relation to FIGS. 8-11. The anchor driver 570 includes an axial through hole 596 to allow passage of the set screw 416 and the set screw driver (not shown) for locking the suture 418.

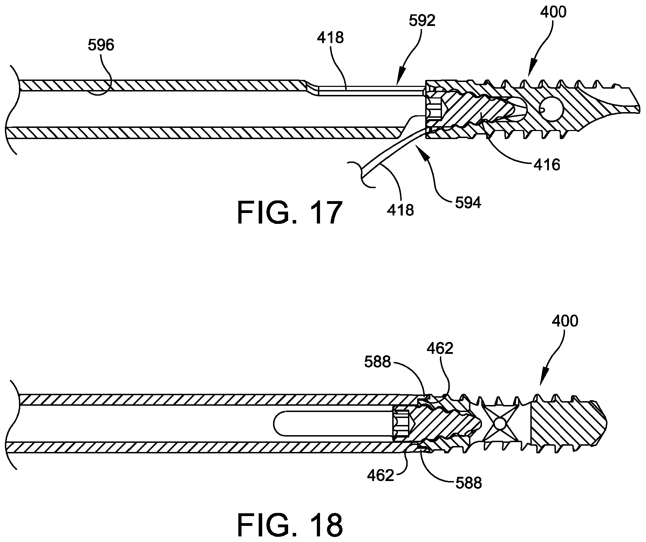

In use, for example, the anchor 400 is driven into a bone by engaging the tabs 588 of the anchor driver 570 with the slots 462 in the anchor, as shown in FIG. 18, and rotating the anchor driver 570. A suture 418 is threaded through the anchor 400 and passed through the clearance 592, 594 outside of the driver 570, if the anchor driver 570 has not already been removed. The suture is tensioned and then the set screw 416 is driven into the anchor 400 to lock the suture. If the anchor driver 570 is still engaged with the anchor 400, the set screw 416 may be inserted through the axial through hole 596 in the anchor driver 570. The torsional force resistance provided by the anchor driver 570 engagement with the slots 462 ensures that forces applied in locking the suture via the set screw 416 do not affect or alter the original insertion alignment of the anchor 400.

### Constructs and Operative Sequences

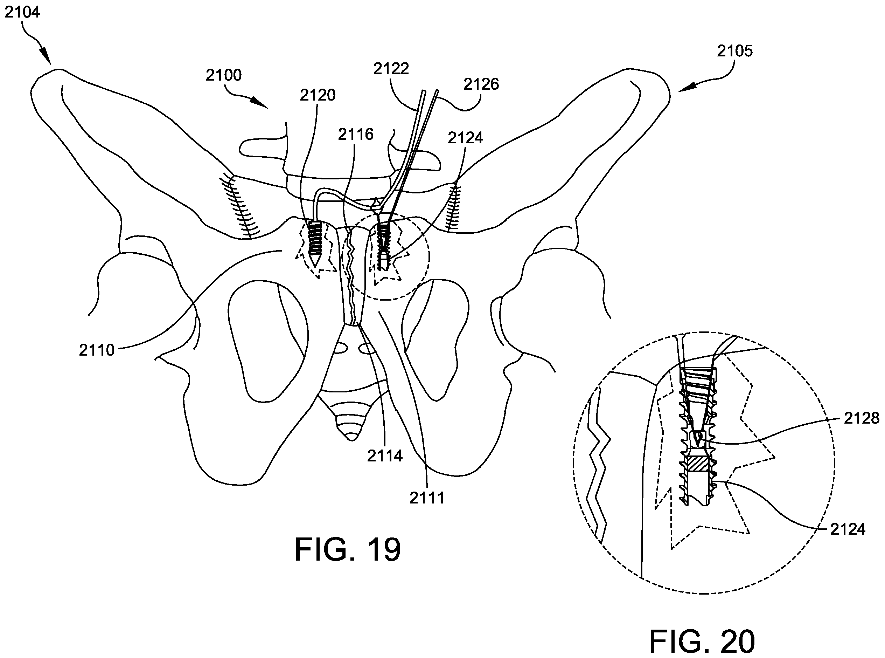

FIGS. 19-26 illustrate a number of examples of embodiments of the disclosed devices in use to stabilize or reinforce ligaments and/or bone fractures. In the example of FIGS. 19-22, the exemplary devices and methods are shown in use in a stabilization construct reinforcing the pubic symphysis 2114 to stabilize the pelvis 2100 after an anterior-posterior compression fracture 2116.

Referring to FIGS. 19 and 20, a first anchor 2120 is inserted into the pubis 2110 of a first hip bone 2104 from superior to inferior. A flexible strand 2122 is connected to the first anchor 2120 and extends from the trailing end of the first anchor. In the embodiments disclosed herein, the flexible strand may be a suture, a suture tape, a cable or another suitable synthetic flexible strand. In the example of FIGS. 19-22, the flexible strand 2122 is a braided high strength suture tape.

A second anchor 2124 such as, for example, anchors 400 or 500 discussed above in relation to FIGS. 5-13, is inserted into the pubis 2111 of a second hip bone 2105 from superior to inferior. The position of the second anchor 2124 is shown via the broken-out section view of FIG. 20, in which the second anchor 2124 is shown in cross section. A suture threader or passer 2126 extends into the proximal or trailing end of the second anchor 2124, around a return feature 2128, and out the proximal or trailing end of the second anchor 2124. The flexible strand 2122 is then threaded through a loop of the passer 2126.

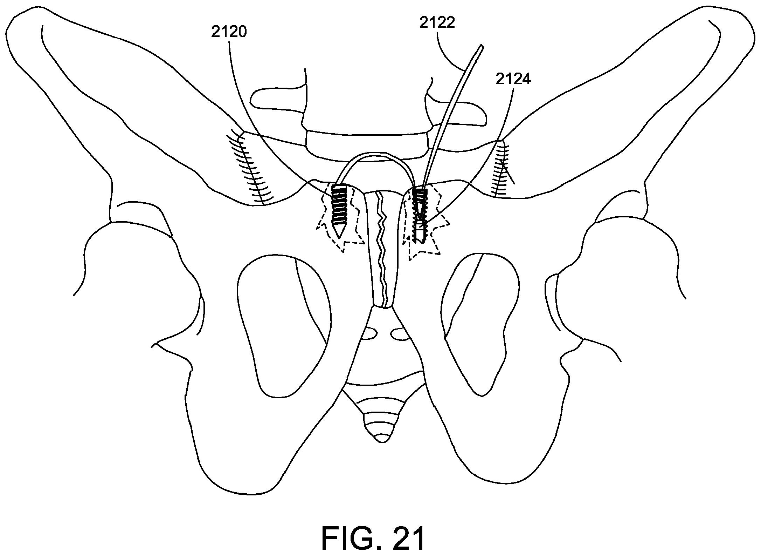

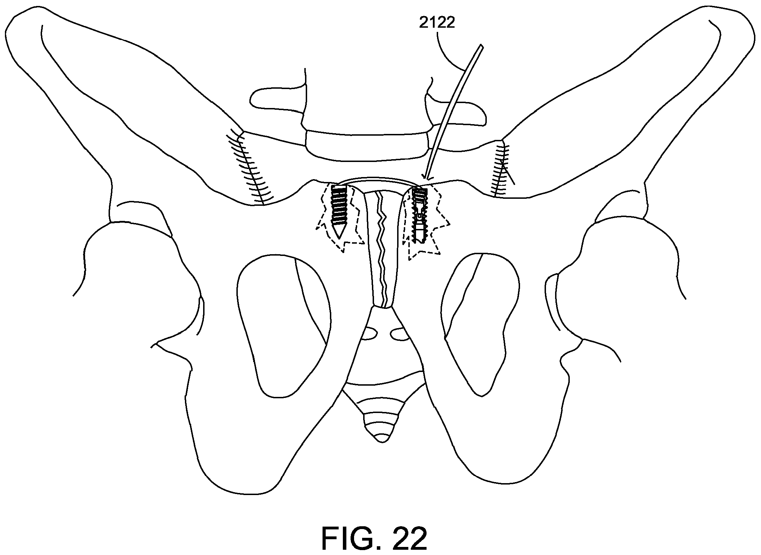

Referring to FIG. 21, the passer 2126 has been withdrawn from the proximal end of the second anchor 2124 to pass the end of the flexible strand 2122 into the trailing end or proximal end of the second anchor 2124, around the return feature 2128, and out the trailing end of the second anchor 2124. A tension instrument may then be used to pull the flexible strand 2122 to tension the portion of the flexible strand 2122 extending between the anchors 2120 and 2124 and reduce the pubic symphysis. The flexible strand 2122 may then be locked relative to the second anchor 2124 to maintain the reduction in any appropriate manner. In this regard, a set screw may be advanced through a central cannulation in the tension instrument before a set screw driver is advanced through the central cannulation in the tension instrument and used to thread the set screw into the internal threads formed in the second anchor 2124 to lock the flexible strand 2122 relative to the second anchor at the desired tension. The tension instrument may then be released and removed. Once the flexible strand is locked, the excess length of the flexible strand 2122 may be trimmed, as shown in FIG. 22.

A novel repair construct according to examples of the invention has been shown with anchors inserted into the superior portion of the pubis to create a superior tension band. However, it will be understood that the anchors may be inserted in other orientations and at other locations. For example, the anchors may be inserted in the superior portion of the pubis but directed anterior to posterior or at some angle between superior-inferior and anterior-posterior. Likewise, the anchors may be inserted inferior to the positions shown in the illustrative example of FIGS. 19-22 to create an anterior tension band midway between the superior and inferior aspects of the pubis. Multiple bands may be applied at various levels as needed to achieve stability. Reinforcement/repair of the sacroiliac joint may be combined with the anterior reinforcement.

While FIGS. 19-22 detail an exemplary method and associated devices for forming a fracture repair construct, similar methods and devices may be used to form ligament reinforcement constructs such as, for example, within the human ankle 100 detailed in FIGS. 2-4 above.

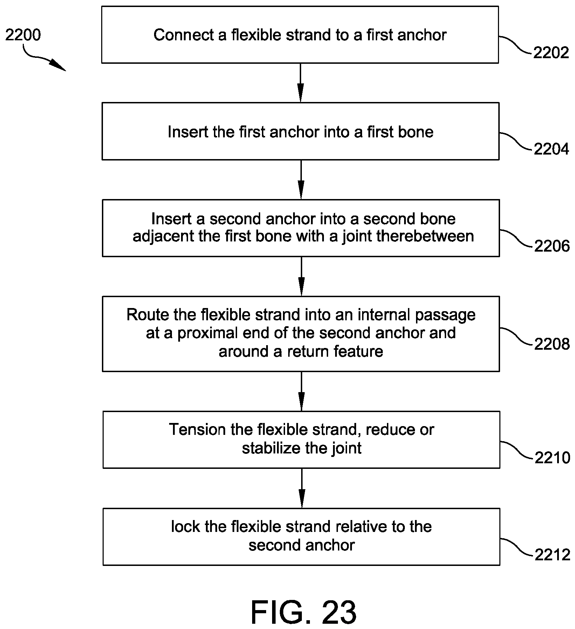

In another exemplary method, and with reference to FIG. 23, there may be provided a method of at least one of reducing or stabilizing a joint. The method may include connecting 2202 a flexible strand to a first anchor. The method may include inserting 2204 the first anchor into a first bone. The method may include inserting 2206 a second anchor into a second bone adjacent the first bone with a joint there between. The method may include routing 2208 the flexible strand into an internal passage at the proximal end of the second anchor and around a return feature. The method may include tensioning 2210 the flexible strand to reduce or stabilize the joint. The method may include locking 2212 the suture to the flexible strand. In an embodiment, the routing of the flexible strand into the internal passage at the proximal end of the second anchor and around the return feature may include exiting the flexible strand from the internal passage out of the proximal end of the second anchor. In another embodiment, the routing of the flexible strand into the internal passage at the proximal end of the second anchor and around the return feature may include exiting the flexible strand from the internal passage out of a side aperture formed through the second anchor. In another embodiment, the routing of the flexible strand into the internal passage at the proximal end of the second anchor and around the return feature may include exiting the flexible strand from the internal passage out of a distal end of the second anchor. Locking the suture to the flexible strand may include a reversible configuration so as to further allow unlocking the flexible strand relative to the second anchor. Locking the suture to the flexible strand may include applying a screw.

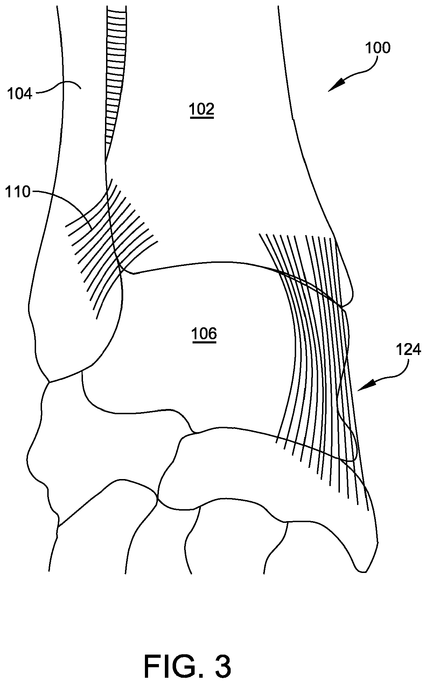

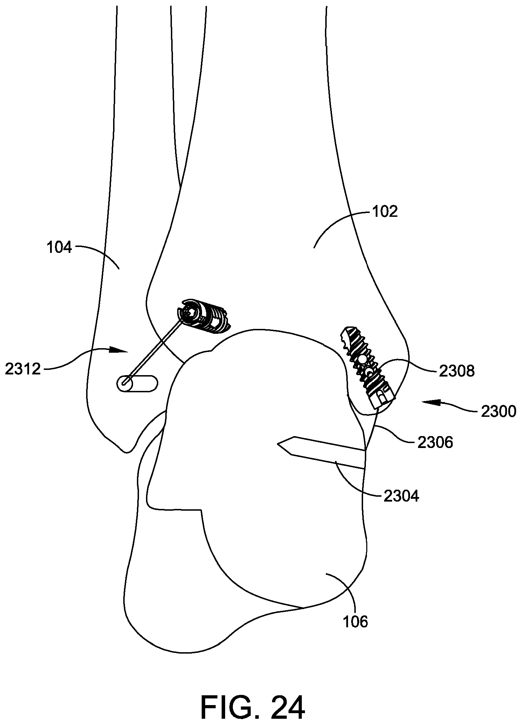

FIG. 24 illustrates a first ligament reinforcement construct 2300 for reinforcing the deltoid ligament complex 124 (FIG. 3). In this construct embodiment, a suture anchor 2304 with a trailing suture 2306 has been has been placed in the talus 106. A second suture returning and locking anchor 2308 such as, for example, one of anchors 400 or 500 discussed above in relation to FIGS. 5-13 has been placed in the medial malleolus of the tibia 102. In this example, the suture 2306 trailing from the anchor 2304 is threaded through the second anchor 2308, tensioned, e.g., manually or using a tension instrument, and locked or affixed with a set screw as discussed above in relation to FIGS. 8-11. FIG. 24 also shows a separate and similar ligament reinforcement construct 2312 for reinforcement of, for example, the posterior inferior tibiofibular ligament (PITFL) 112 or the anterior inferior tibiofibular ligament (AITFL) 110 (FIGS. 2-4).

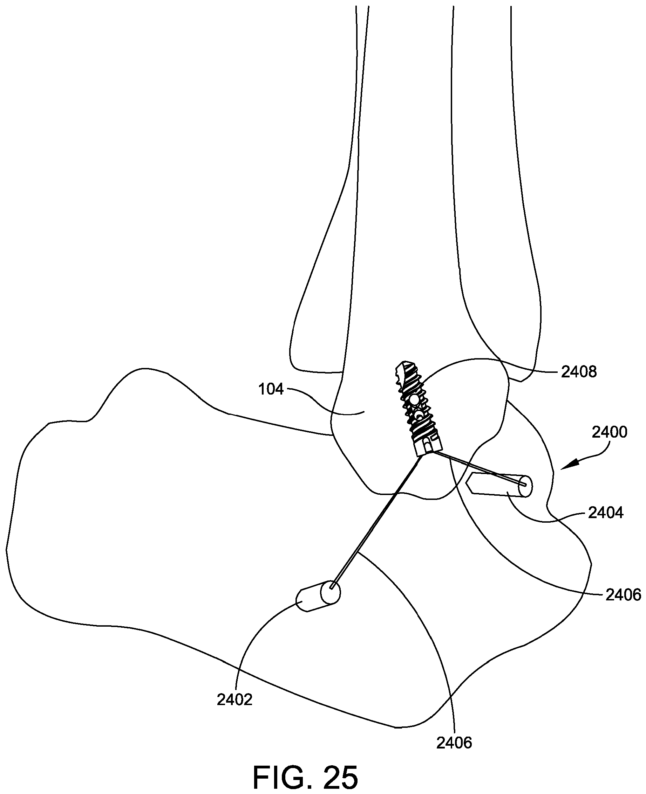

FIG. 25 illustrates another reinforcement construct 2400 for reinforcing, for example, the anterior talofibular ligament (ATFL) 120, the posterior talofibular ligament (PTFL) 122, or other ligaments. In the example of FIG. 25, two suture anchors 2402, 2404 have been placed with suture 2406 to reinforce two separate ligaments, but a single suture returning and locking anchor 2408 such as, for example, the anchors 400 or 500 discussed above in relation to FIGS. 5-13, has been placed in the fibula 104 to secure both ligaments to a common attachment or fixation point.

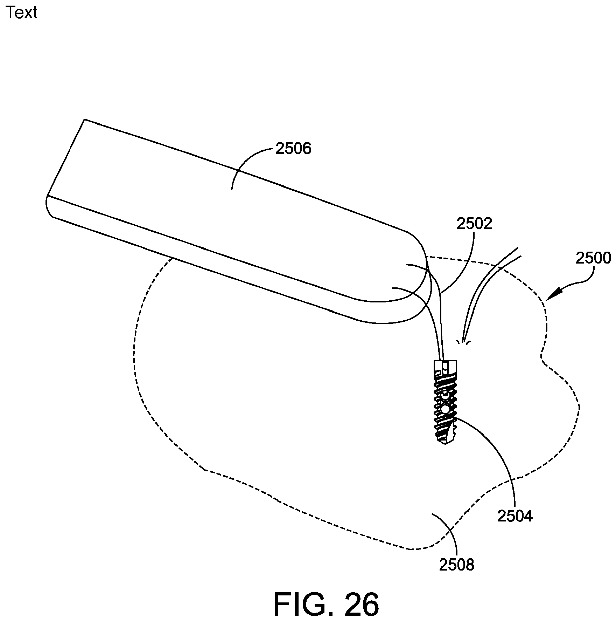

Beyond the reinforcement and repair constructs discussed above, suture ends from embodiments of the returning and locking anchors discussed above may be used to attach other soft human or allograft tissues. FIG. 26 illustrates an exemplary attachment construct 2500 in which a suture returning and locking anchor 2504 such as, for example, one of the anchors 400 or 500 discussed above in relation to FIGS. 5-13 has been employed for the purpose of soft tissue repair or tendon reattachment. In this embodiment, a flexible strand such as a suture 2502 is passed through a tendon 2506 (e.g., Achilles tendon, rotator cuff tendon, etc.), allograft, or other soft tissue before being threaded through the anchor 2500, which is affixed with an appropriate bone 2508, tensioned, and locked, as discussed above.

Notably, any combination of ordinary suture anchors, suture returning anchors, suture locking anchors, suture returning and locking anchors, and any number of sutures per anchor may be combined to produce a variety of constructs with one-to-one or many-to-one relationships.

Although the above embodiments have been described in language that is specific to certain structures, elements, compositions, and methodological steps, it is to be understood that the technology defined in the appended claims is not necessarily limited to the specific structures, elements, compositions and/or steps described. Rather, the specific aspects and steps are described as forms of implementing the technology.

## Claims

1. A knotless returning and locking system for bone fracture stabilization and soft tissue repair and reinforcement, comprising:
a returning and locking anchor (400, 500) having a body (402, 502) with a proximal end (404, 504), a distal end (406, 506), and defining a longitudinal axis (408, 508), the body (402, 502) forming an internal passage (441, 541) and a return feature (413, 513), the internal passage (441, 541) having a threaded receiver (430, 530) located at the proximal end (404, 504) of the body (402, 502) and including a proximal portion, a mid portion, and a distal portion, the return feature (413, 513) located distal to the threaded receiver (430, 530) and in communication with the internal passage (441, 541); and
a threaded set screw (416, 516) having a proximal portion, a mid portion, and a distal portion, **characterized in that** the threaded set screw (416, 516) is configured for rotational insertion into the threaded receiver (430, 530) to achieve a progressively increasing interference fit about a flexible synthetic strand (418, 518) passing between the proximal portions and the mid portions of the threaded receiver (430, 530) and the threaded set screw (416, 516) and a progressively decreasing interference fit about the flexible synthetic strand (418, 518) passing between the mid portions and the distal portions of the threaded receiver (430, 530) and the threaded set screw (416, 516), wherein the progressively increasing interference fit and the progressively decreasing interference fit combine to provide a locking feature that reversibly secures the flexible synthetic strand (418, 518) in relation to the returning and locking anchor (400, 500).

2. The knotless returning and locking system of claim 1, further comprising a flexible synthetic strand (418, 518), wherein the flexible synthetic strand (418, 518) enters the returning and locking anchor (400, 500) through the internal passage (441, 541) at the proximal end (404, 504) of the body (402, 502), routes around the return feature (413, 513), and exits the returning and locking anchor through the internal passage (441, 541) at the proximal end (404, 504) of the body (402, 502);
optionally wherein the return feature (413, 513) comprises:
a first distal hole (438, 538) positioned transverse to the longitudinal axis (408, 508);
a second proximal hole (444, 560) positioned transverse to the longitudinal axis (408, 508), each of the first distal (438, 538) and the second proximal (444, 560) holes in communication with the internal passage (441, 541) and forming opposed openings on opposite sides of the body (402, 502); and
a margin (440) separating the first distal (438, 538) and the second proximal (444, 560) holes, wherein:
after the flexible synthetic strand (418, 518) enters the returning and locking anchor through the internal passage (441, 541) at the proximal end (404, 504) of the body (402, 502), the flexible synthetic strand exits one of the opposed openings of the second proximal hole (444, 560), passes through the first distal hole(438, 538), and enters another of the openings of the second proximal hole (444, 560), before exiting through the internal passage (441, 541) at the proximal end (404, 504) of the body (402, 502) such that the flexible synthetic strand (418, 518) is routed around the margin (440);
further optionally wherein the body (502) of the returning and locking anchor (500) further comprises a removable return insert (550) having a longitudinal body (552) centered about the longitudinal axis (508) and sized for insertion into the internal passage (541) from the distal end (506) of the body (502) of the returning and locking anchor (500), wherein the first distal hole and the margin are incorporated into removable return insert (550).

3. The knotless returning and locking system of claim 1, further comprising a flexible synthetic strand (418, 518), wherein the flexible synthetic strand (418, 518) enters the returning and locking anchor (400, 500) through the internal passage (441, 541) at the proximal end (404, 504) of the body (402, 502), routes around the return feature (413, 513), and exits the returning and locking anchor (400, 500) through a sidewall of the body (402, 502) at a location distal to the proximal end (404, 504) of the body (402, 502);
optionally wherein the return feature (413, 513) comprises a proximal edge of a hole through the sidewall of the body (402, 502).

4. The knotless returning and locking system of claim 1, further comprising a flexible synthetic strand (418, 518), wherein the flexible synthetic strand (418, 518) enters the returning and locking anchor (400, 500) through the internal passage (441, 541) at the proximal end (404, 504) of the body (402, 502), and exits through a distal end (406, 506) of the body (402, 502) in opposition to the proximal end (404, 504) of the body (402, 502).

5. The knotless returning and locking system of claim 1, wherein:
the proximal portion of the set screw (416, 516) tapers at a proximal set screw angle and the proximal portion of the receiver (430, 530) tapers at an opposing proximal receiver angle, the proximal set screw angle diverging from the opposing proximal receiver angle;
the mid portion of the set screw (416, 516) tapers at a mid set screw angle and the mid portion of the receiver (430, 530) tapers at an opposing mid receiver angle, the mid set screw angle approximating the opposing mid receiver angle;
the distal portion of the set screw (416, 516) tapers at a distal set screw angle and the distal portion of the receiver (430, 530) tapers at an opposing distal receiver angle, the distal set screw angle diverging from the opposing distal receiver angle, such that when the set screw (416, 516) is rotationally inserted into the receiver (430, 530), a compression force applied to the flexible synthetic strand (418, 518) gradually increases proximally leading into and gradually decreases distally leading out of a region of alignment between the mid portions of the set screw (416, 516) and the receiver (430, 530).

6. The knotless returning and locking system of claim 1, wherein the threaded receiver (430, 530) and the threaded set screw (416, 516) each comprise knuckle threads.

7. The knotless returning and locking system of claim 1, further comprising:
a flexible synthetic strand (2122) having first and second opposing ends;
a first fixation comprising a first anchor (2120) secured at the first end of the flexible synthetic strand (2122);
a second fixation secured at the second end of the flexible synthetic strand (2122), the second fixation comprising said returning and locking anchor (400, 500, 2124) inserted into a bone portion (2111), wherein
the second end of the flexible synthetic strand (2212) enters the returning and locking anchor (400, 500, 2124) through the internal passage (441, 541) at the proximal end (404, 504) of the body (402, 502);
the flexible synthetic strand (2212) is tensioned between the first and the second fixations; and
the second end of the flexible synthetic strand is locked relative to the returning and locking anchor (400, 500, 2124) resulting in a continuous, uninterrupted length of the flexible synthetic strand extending externally across the joint between the first and the second fixations.

8. The knotless returning and locking system of claim 7, wherein the flexible synthetic strand (2122) enters the locking anchor (400, 500, 2124) through the axial passage at the proximal end of the body, routes around the return feature (413, 513, 2128), and exits the returning and locking anchor (400, 500, 2124) through a sidewall of the body at a location distal to the proximal end of the body.

9. The knotless returning and locking system of claim 7, wherein the flexible synthetic strand (2122) enters the returning and locking anchor (400, 500, 2124) through the internal passage (441, 541) at the proximal end of the body, and exits through a distal end of the body in opposition to the proximal end of the body.

10. The knotless returning and locking system of claim 7, wherein the second end of the flexible synthetic strand is locked via a threaded set screw (416, 516) that is rotationally inserted into the threaded receiver (430, 530).

11. The knotless returning and locking system of claim 7, wherein the second end of the flexible synthetic strand is trimmed flush with the proximal end of the body of the returning and locking anchor (400, 500, 2124).

12. The knotless returning and locking system of claim 7, wherein the first fixation comprises the first anchor (2120) inserted into another bone portion (2110) such that the construct provides bone fracture stabilization or ligament reinforcement.

13. The knotless returning and locking system of claim 7, wherein the first fixation comprises the first end of the flexible synthetic strand passed through a soft tissue portion such that the construct provides soft tissue repair or tendon attachment; or
wherein the first fixation further comprises multiple additional fixations, each of the multiple additional fixations coupled in tension to the returning and locking anchor via an additional flexible synthetic strand for each of the multiple additional fixations.

## Patentansprüche

1. Knotenloses Rückführ- und Verriegelungssystem für Knochenfrakturstabilisierung und Weichgewebereparatur und -verstärkung, umfassend:
einen Rückführ- und Verriegelungsanker (400, 500), der einen Körper (402, 502) mit einem proximalen Ende (404, 504) und einem distalen Ende (406, 506) aufweist und eine Längsachse (408, 508) definiert, wobei der Körper (402, 502) einen inneren Durchgang (441, 541) und ein Rückführmerkmal (413, 513) bildet, wobei der innere Durchgang (441, 541) einen Gewindeaufnehmer (430, 530) aufweist, der an dem proximalen Ende (404, 504) des Körpers (402, 502) angeordnet ist und einen proximalen Abschnitt, einen mittleren Abschnitt und einen distalen Abschnitt aufweist, wobei das Rückführmerkmal (413, 513) distal von dem Gewindeaufnehmer (430, 530) und in Verbindung mit dem inneren Durchgang (441, 541) angeordnet ist, und
eine Gewindestellschraube (416, 516) mit einem proximalen Abschnitt, einem mittleren Abschnitt und einem distalen Abschnitt, **dadurch gekennzeichnet, dass** die Gewindestellschraube (416, 516) für die Dreheinführung in den Gewindeaufnehmer (430, 530) ausgestaltet ist, um eine fortschreitend zunehmende Presspassung um einen zwischen den proximalen Abschnitten und den mittleren Abschnitten des Gewindeaufnehmers (430, 530) und der Gewindestellschraube (416, 516) hindurchgehenden flexiblen synthetischen Strang (418, 518) herum und eine fortschreitend abnehmende Presspassung um den zwischen den mittleren Abschnitten und den distalen Abschnitten des Gewindeaufnehmers (430, 530) und der Gewindestellschraube (416, 516) hindurchgehenden flexiblen synthetischen Strang (418, 518) herum zu erzielen, wobei die fortschreitend zunehmende Presspassung und die fortschreitend abnehmende Presspassung zusammenwirken, um ein Verriegelungsmerkmal bereitzustellen, das den flexiblen synthetischen Strang (418, 518) in Bezug auf den Rückführ- und Verriegelungsanker (400, 500) reversibel befestigt.

2. Knotenloses Rückführ- und Verriegelungssystem nach Anspruch 1, ferner umfassend einen flexiblen synthetischen Strang (418, 518), wobei der flexible synthetische Strang (418, 518) durch den inneren Durchgang (441, 541) an dem proximalen Ende (404, 504) des Körpers (402, 502) in den Rückführ- und Verriegelungsanker (400, 500) eintritt, um das Rückführmerkmal (413, 513) herumgeht und durch den inneren Durchgang (441, 541) an dem proximalen Ende (404, 504) des Körpers (402, 502) aus dem Rückführ- und Verriegelungsanker austritt,
optional wobei das Rückführmerkmal (413, 513) Folgendes umfasst:
ein erstes, distales Loch (438, 538), das quer zu der Längsachse (408, 508) positioniert ist,
ein zweites, proximales Loch (444, 560), das quer zu der Längsachse (408, 508) positioniert ist, wobei das erste, distale (438, 538) und das zweite, proximale Loch (444, 560) jeweils in Verbindung mit dem inneren Durchgang (441, 541) stehen und gegenüberliegende Öffnungen an gegenüberliegenden Seiten des Körpers (402, 502) bilden, und
einen Rand (440), der das erste, distale (438, 538) und das zweite, proximale Loch (444, 560) trennt, wobei:
nachdem der flexible synthetische Strang (418, 518) durch den inneren Durchgang (441, 541) an dem proximalen Ende (404, 504) des Körpers (402, 502) in den Rückführ- und Verriegelungsanker eingetreten ist, der flexible synthetische Strang aus einer der gegenüberliegenden Öffnungen des zweiten, proximalen Lochs (444, 560) austritt, durch das erste, distale Loch (438, 538) geht und in eine andere der Öffnungen des zweiten, proximalen Lochs (444, 560) eintritt, bevor er durch den inneren Durchgang (441, 541) an dem proximalen Ende (404, 504) des Körpers (402, 502) austritt, so dass der flexible synthetische Strang (418, 518) um den Rand (440) herumgeht,
ferner optional wobei der Körper (502) des Rückführ- und Verriegelungsankers (500) ferner einen entfernbaren Rückführeinsatz (550) umfasst, der einen länglichen Körper (552) aufweist, der um die Längsachse (508) zentriert ist und zur Einführung in den inneren Durchgang (541) von dem distalen Ende (506) des Körpers (502) des Rückführ- und Verriegelungsankers (500) her bemessen ist, wobei das erste distale Loch und der Rand in den entfernbaren Rückführeinsatz (550) eingegliedert sind.

3. Knotenloses Rückführ- und Verriegelungssystem nach Anspruch 1, ferner umfassend einen flexiblen synthetischen Strang (418, 518), wobei der flexible synthetische Strang (418, 518) durch den inneren Durchgang (441, 541) an dem proximalen Ende (404, 504) des Körpers (402, 502) in den Rückführ- und Verriegelungsanker (400, 500) eintritt, um das Rückführmerkmal (413, 513) herumgeht und durch eine Seitenwand des Körpers (402, 502) an einer distal zu dem proximalen Ende (404, 504) des Körpers (402, 502) liegenden Stelle aus dem Rückführ- und Verriegelungsanker (400, 500) austritt,
optional wobei das Rückführmerkmal (413, 513) eine proximale Kante eines Lochs durch die Seitenwand des Körpers (402, 502) umfasst.

4. Knotenloses Rückführ- und Verriegelungssystem nach Anspruch 1, ferner umfassend einen flexiblen synthetischen Strang (418, 518), wobei der flexible synthetische Strang (418, 518) durch den inneren Durchgang (441, 541) an dem proximalen Ende (404, 504) des Körpers (402, 502) in den Rückführ- und Verriegelungsanker (400, 500) eintritt und durch ein distales Ende (406, 506) des Körpers (402, 502) gegenüber dem proximalen Ende (404, 504) des Körpers (402, 502) austritt.

5. Knotenloses Rückführ- und Verriegelungssystem nach Anspruch 1, wobei:
sich der proximale Abschnitt der Stellschraube (416, 516) in einem proximalen Stellschraubenwinkel verjüngt und sich der proximale Abschnitt des Aufnehmers (430, 530) in einem entgegengesetzten proximalen Aufnehmerwinkel verjüngt, wobei der proximale Stellschraubenwinkel von dem entgegengesetzten proximalen Aufnehmerwinkel divergiert,
sich der mittlere Abschnitt der Stellschraube (416, 516) in einem mittleren Stellschraubenwinkel verjüngt und sich der mittlere Abschnitt des Aufnehmers (430, 530) in einem entgegengesetzten mittleren Aufnehmerwinkel verjüngt, wobei der mittlere Stellschraubenwinkel den entgegengesetzten mittleren Aufnehmerwinkel approximiert,
sich der distale Abschnitt der Stellschraube (416, 516) in einem distalen Stellschraubenwinkel verjüngt und sich der distale Abschnitt des Aufnehmers (430, 530) in einem entgegengesetzten distalen Aufnehmerwinkel verjüngt, wobei der distale Stellschraubenwinkel von dem entgegengesetzten distalen Aufnehmerwinkel divergiert, so dass wenn die Stellschraube (416, 516) drehungsmäßig in den Aufnehmer (430, 530) eingeführt wird, eine auf den flexiblen synthetischen Strang (418, 518) ausgeübte Druckkraft in einen Ausrichtungsbereich zwischen den mittleren Abschnitten der Stellschraube (416, 516) und des Aufnehmers (430, 530) hineinführend allmählich proximal zunimmt und aus diesem Bereich herausführend allmählich distal abnimmt.

6. Knotenloses Rückführ- und Verriegelungssystem nach Anspruch 1, wobei der Gewindeaufnehmer (430, 530) und die Gewindestellschraube (416, 516) jeweils ein Rundgewinde umfassen.

7. Knotenloses Rückführ- und Verriegelungssystem nach Anspruch 1, ferner umfassend:
einen flexiblen synthetischen Strang (2122) mit einem ersten und einem zweiten Ende, die sich gegenüberliegen,
ein erste Fixierung, die einen ersten Anker (2120) umfasst und an dem ersten Ende des flexiblen synthetischen Strangs (2122) befestigt ist,
ein zweite Fixierung, die an dem zweiten Ende des flexiblen synthetischen Strangs (2122) befestigt ist, wobei die zweite Fixierung den in einen Knochenabschnitt (2111) eingeführten Rückführ- und Verriegelungsanker (400, 500, 2124) umfasst, wobei
das zweite Ende des flexiblen synthetischen Strangs (2122) durch den inneren Durchgang (441, 541) an dem proximalen Ende (404, 504) des Körpers (402, 502) in den Rückführ- und Verriegelungsanker (400, 500, 2124) eintritt,
der flexible synthetische Strang (2122) zwischen der ersten und der zweiten Fixierung gespannt ist und
das zweite Ende des flexiblen synthetischen Strangs bezüglich des Rückführ- und Verriegelungsankers (400, 500, 2124) verriegelt ist, was zu einer kontinuierlichen ununterbrochenen Länge des flexiblen synthetischen Strangs führt, der sich extern über das Gelenk zwischen der ersten und der zweiten Fixierung erstreckt.

8. Knotenloses Rückführ- und Verriegelungssystem nach Anspruch 7, wobei der flexible synthetische Strang (2122) durch den axialen Durchgang an dem proximalen Ende des Körpers in den Verriegelungsanker (400, 500, 2124) eintritt, um das Rückführmerkmal (413, 513, 2128) herumgeht und durch eine Seitenwand des Körpers an einer distal zu dem proximalen Ende des Körpers liegenden Stelle aus dem Rückführ- und Verriegelungsanker (400, 500, 2124) austritt.

9. Knotenloses Rückführ- und Verriegelungssystem nach Anspruch 7, wobei der flexible synthetische Strang (2122) durch den inneren Durchgang (441, 541) an dem proximalen Ende des Körpers in den Rückführ- und Verriegelungsanker (400, 500, 2124) eintritt und durch ein distales Ende des Körpers gegenüber dem proximalen Ende des Körpers austritt.

10. Knotenloses Rückführ- und Verriegelungssystem nach Anspruch 7, wobei das zweite Ende des flexiblen synthetischen Strangs über eine Gewindestellschraube (416, 516), die drehungsmäßig in den Gewindeaufnehmer (430, 530) eingeführt ist, verriegelt ist.

11. Knotenloses Rückführ- und Verriegelungssystem nach Anspruch 7, wobei das zweite Ende des flexiblen synthetischen Strangs bündig mit dem proximalen Ende des Körpers des Rückführ- und Verriegelungsankers (400, 500, 2124) zugeschnitten ist.

12. Knotenloses Rückführ- und Verriegelungssystem nach Anspruch 7, wobei die erste Fixierung den ersten Anker (2120) umfasst, der in einen anderen Knochenabschnitt (2110) eingeführt ist, so dass das Gebilde für Knochenfrakturstabilisierung oder Bänderverstärkung sorgt.

13. Knotenloses Rückführ- und Verriegelungssystem nach Anspruch 7, wobei die erste Fixierung das erste Ende des flexiblen synthetischen Strangs umfasst, das durch einen Weichgewebeabschnitt geführt worden ist, so dass das Gebilde für Weichgewebereparatur oder Sehnenbefestigung sorgt, oder
wobei die erste Fixierung ferner mehrere zusätzliche Fixierungen umfasst, wobei jede der mehreren zusätzlichen Fixierungen über einen zusätzlichen flexiblen synthetischen Strang für jede der mehreren Fixierungen mit Spannung an den Rückführ- und Verriegelungsanker gekoppelt ist.

## Revendications

1. Système de retour et de blocage sans noeuds pour la stabilisation d'une fracture osseuse et la réparation et le renforcement d'un tissu mou, comprenant :
une ancre de retour et de blocage (400, 500) ayant un corps (402, 502) avec une extrémité proximale (404, 504), une extrémité distale (406, 506), et définissant un axe longitudinal (408, 508), le corps (402, 502) formant un passage interne (441, 541) et un élément de retour (413, 513), le passage interne (441, 541) ayant un récepteur fileté (430, 530) situé à l'extrémité proximale (404, 504) du corps (402, 502) et comportant une partie proximale, une partie centrale et une partie distale, l'élément de retour (413, 513) étant situé distalement par rapport au récepteur fileté (430, 530) et en communication avec le passage interne (441, 541) ; et
une vis de pression filetée (416, 516) ayant une partie proximale, une partie centrale et une partie distale, **caractérisé en ce que** la vis de pression filetée (416, 516) est configurée pour être insérée par rotation dans le récepteur fileté (430, 530) pour réaliser un ajustement de plus en plus serré autour d'un fil synthétique souple (418, 518) passant entre les parties proximales et les parties centrales du récepteur fileté (430, 530) et de la vis de pression filetée (416, 516) et un ajustement de moins en moins serré autour du fil synthétique souple (418, 518) passant entre les parties centrales et les parties distales du récepteur fileté (430, 530) et de la vis de pression filetée (416, 516), dans lequel l'ajustement de plus en plus serré et l'ajustement de moins en moins serré se combinent pour fournir un élément de blocage qui fixe de façon réversible le fil synthétique souple (418, 518) par rapport à l'ancre de retour et de blocage (400, 500).

2. Système de retour et de blocage sans noeuds de la revendication 1, comprenant en outre un fil synthétique souple (418, 518), dans lequel le fil synthétique souple (418, 518) entre dans l'ancre de retour et de blocage (400, 500) par le passage interne (441, 541) à l'extrémité proximale (404, 504) du corps (402, 502), chemine autour de l'élément de retour (413, 513), et sort de l'ancre de retour et de blocage par le passage interne (441, 541) à l'extrémité proximale (404, 504) du corps (402, 502) ;
éventuellement dans lequel l'élément de retour (413, 513) comprend :
un premier trou distal (438, 538) positionné transversalement à l'axe longitudinal (408, 508) ;
un deuxième trou proximal (444, 560) positionné transversalement à l'axe longitudinal (408, 508), le premier trou distal (438, 538) et le deuxième trou proximal (444, 560) étant chacun en communication avec le passage interne (441, 541) et formant chacun des ouvertures opposées sur des côtés opposés du corps (402, 502) ; et
une bordure (440) séparant le premier trou distal (438, 538) et le deuxième trou proximal (444, 560), dans lequel :
après que le fil synthétique souple (418, 518) est entré dans l'ancre de retour et de blocage par le passage interne (441, 541) à l'extrémité proximale (404, 504) du corps (402, 502), le fil synthétique souple sort par une des ouvertures opposées du deuxième trou proximal (444, 560), passe par le premier trou distal (438, 538), et entre dans une autre des ouvertures du deuxième trou proximal (444, 560), avant de sortir par le passage interne (441, 541) à l'extrémité proximale (404, 504) du corps (402, 502), de telle sorte que le fil synthétique souple (418, 518) est acheminé autour de la bordure (440) ;
éventuellement dans lequel, en outre, le corps (502) de l'ancre de retour et de blocage (500) comprend en outre une pièce rapportée de retour amovible (550) ayant un corps longitudinal (552) centré autour de l'axe longitudinal (508) et dimensionné pour être inséré dans le passage interne (541) depuis l'extrémité distale (506) du corps (502) de l'ancre de retour et de blocage (500), dans lequel le premier trou distal et la bordure sont incorporés dans la pièce rapportée de retour amovible (550).

3. Système de retour et de blocage sans noeuds de la revendication 1, comprenant en outre un fil synthétique souple (418, 518), dans lequel le fil synthétique souple (418, 518) entre dans l'ancre de retour et de blocage (400, 500) par le passage interne (441, 541) à l'extrémité proximale (404, 504) du corps (402, 502), chemine autour de l'élément de retour (413, 513), et sort de l'ancre de retour et de blocage (400, 500) par une paroi latérale du corps (402, 502) à un emplacement distal par rapport à l'extrémité proximale (404, 504) du corps (402, 502) ;
éventuellement dans lequel l'élément de retour (413, 513) comprend un bord proximal d'un trou à travers la paroi latérale du corps (402, 502).

4. Système de retour et de blocage sans noeuds de la revendication 1, comprenant en outre un fil synthétique souple (418, 518), dans lequel le fil synthétique souple (418, 518) entre dans l'ancre de retour et de blocage (400, 500) par le passage interne (441, 541) à l'extrémité proximale (404, 504) du corps (402, 502), et sort par une extrémité distale (406, 506) du corps (402, 502) à l'opposé de l'extrémité proximale (404, 504) du corps (402, 502).

5. Système de retour et de blocage sans noeuds de la revendication 1, dans lequel :
la partie proximale de la vis de pression (416, 516) s'effile à un angle proximal de vis de pression et la partie proximale du récepteur (430, 530) s'effile à un angle proximal de récepteur opposé, l'angle proximal de vis de pression divergeant de l'angle proximal de récepteur opposé ;
la partie centrale de la vis de pression (416, 516) s'effile à un angle central de vis de pression et la partie centrale du récepteur (430, 530) s'effile à un angle central de récepteur opposé, l'angle central de vis de pression se rapprochant de l'angle central de récepteur opposé ;
la partie distale de la vis de pression (416, 516) s'effile à un angle distal de vis de pression et la partie distale du récepteur (430, 530) s'effile à un angle distal de récepteur opposé, l'angle distal de vis de pression divergeant de l'angle distal de récepteur opposé, de telle sorte que, quand la vis de pression (416, 516) est insérée par rotation dans le récepteur (430, 530), une force de compression appliquée au fil synthétique souple (418, 518) augmente progressivement en progressant proximalement vers l'intérieur et diminue progressivement en progressant distalement vers l'extérieur d'une région d'alignement entre les parties centrales de la vis de pression (416, 516) et du récepteur (430, 530).

6. Système de retour et de blocage sans noeuds de la revendication 1, dans lequel le récepteur fileté (430, 530) et la vis de pression filetée (416, 516) comprennent chacun des filets ronds.

7. Système de retour et de blocage sans noeuds de la revendication 1, comprenant en outre :
un fil synthétique souple (2122) ayant des première et deuxième extrémités opposées ;
une première fixation comprenant une première ancre (2120) fixée à la première extrémité du fil synthétique souple (2122) ;
une deuxième fixation fixée à la deuxième extrémité du fil synthétique souple (2122), la deuxième fixation comprenant ladite ancre de retour et de blocage (400, 500, 2124) insérée dans une partie d'os (2111), dans lequel :
la deuxième extrémité du fil synthétique souple (2122) entre dans l'ancre de retour et de blocage (400, 500, 2124) par le passage interne (441, 541) à l'extrémité proximale (404, 504) du corps (402, 502) ;
le fil synthétique souple (2122) est mis sous tension entre les première et deuxième fixations ; et
la deuxième extrémité du fil synthétique souple est bloquée par rapport à l'ancre de retour et de blocage (400, 500, 2124), ce qui se solde par une longueur continue, ininterrompue du fil synthétique souple s'étendant à l'extérieur d'un bout à l'autre de l'articulation entre les première et deuxième fixations.

8. Système de retour et de blocage sans noeuds de la revendication 7, dans lequel le fil synthétique souple (2122) entre dans l'ancre de blocage (400, 500, 2124) par le passage axial à l'extrémité proximale du corps, chemine autour de l'élément de retour (413, 513, 2128), et sort de l'ancre de retour et de blocage (400, 500, 2124) par une paroi latérale du corps à un emplacement distal par rapport à l'extrémité proximale du corps.

9. Système de retour et de blocage sans noeuds de la revendication 7, dans lequel le fil synthétique souple (2122) entre dans l'ancre de retour et de blocage (400, 500, 2124) par le passage interne (441, 541) à l'extrémité proximale du corps, et sort par une extrémité distale du corps à l'opposé de l'extrémité proximale du corps.

10. Système de retour et de blocage sans noeuds de la revendication 7, dans lequel la deuxième extrémité du fil synthétique souple est bloquée par le biais d'une vis de pression filetée (416, 516) qui est insérée par rotation dans le récepteur fileté (430, 530).

11. Système de retour et de blocage sans noeuds de la revendication 7, dans lequel la deuxième extrémité du fil synthétique souple est coupée au ras de l'extrémité proximale du corps de l'ancre de retour et de blocage (400, 500, 2124).

12. Système de retour et de blocage sans noeuds de la revendication 7, dans lequel la première fixation comprend la première ancre (2120) insérée dans une autre partie d'os (2110) de telle sorte que la construction assure une stabilisation de fracture osseuse ou un renforcement de ligament.

13. Système de retour et de blocage sans noeuds de la revendication 7, dans lequel la première fixation comprend la première extrémité du fil synthétique souple passée à travers une partie de tissu mou de telle sorte que la construction assure une réparation de tissu mou ou une fixation de tendon ; ou
dans lequel la première fixation comprend en outre de multiples fixations supplémentaires, chacune des multiples fixations supplémentaires étant couplée en tension à l'ancre de retour et de blocage par le biais d'un fil synthétique souple supplémentaire pour chacune des multiples fixations supplémentaires.
